# EUROPEAN PATENT SPECIFICATION

(11) **EP 2 918 669 B1**
(45) Date of publication and mention of the grant of the patent: **02.11.2016**
(21) Application number: 15166000.8
(22) Date of filing: 16.01.2009
(51) Int. Cl.: C12M 1/14, C12M 1/00, C12M 1/12, C12M 1/24, C12M 1/04

(54) **LIMITED ACCESS MULTI-LAYER CELL CULTURE SYSTEM**
MEHRSCHICHTIGES ZELLKULTURSYSTEM MIT BEGRENZTEM ZUGANG
SYSTÈME DE CULTURE CELLULAIRE MULTICOUCHE À ACCÈS LIMITÉ

(30) Priority: 25.01.2008 US 62404 P
(43) Date of publication of application: 16.09.2015
(62) Divisional of application: 09704848.2
(73) Proprietor: Corning Incorporated, Corning, New York 14831 (US)
(72) Inventor: Martin, Gregory, Acton, ME 04001 (US); Tanner, Alison, Portsmouth, NH New Hampshire 03801 (US)
(74) Representative: Greene, Simon Kenneth

(56) References cited:
- US-A- 3 409 189
- US-A- 5 484 731
- US-A1- 2005 074 873
- US-A1- 2007 026 516

## Description

### FIELD

The present invention relates generally to a system for containing cells in culture. More specifically, the present invention relates to devices for containing cells in culture which allow for sterile controlled containment and sterile transfer of cells and media into and out of the device.

### BACKGROUND

*In vitro* culturing of cells provides material necessary for research in pharmacology, physiology, and toxicology. Recent advances in pharmaceutical screening techniques allow pharmaceutical companies to rapidly screen vast libraries of compounds against therapeutic targets. These large-scale screening techniques require large numbers of cells grown and maintained *in vitro.* Maintaining these large numbers of cells requires large volumes of cell growth media and reagents and large numbers and types of laboratory cell culture containers and laboratory equipment. This activity is also labor intensive.

Cells are grown in specialized cell culture containers including roller bottles, cell culture dishes and plates, multiwell plates, microtiter plates, common flasks and multi-layered cell growth flasks and vessels. Cells in culture attach to and grow on the bottom surface(s) of the flask, immersed in a suitable sustaining media.

With the advent of cell-based high throughput applications, cell culture vessels have been developed to provide an increased surface area for cell growth while also providing necessary gas exchange. These systems also employ traditional cell culture vessels including common flasks, roller bottles, cell culture dishes, as well as multi-layered cell growth vessels including multi-layer flasks, multi-layer cell culture dishes, bioreactors, cell culture bags and the like, which may include specialized surfaces designed to enhance the cell culture parameters including growth density and differentiation factors.

In addition, cell-based high throughput applications have become automated. Automation permits manipulation of the cell culture vessel much like that performed by the manual operator. Further, flask vessels having multiple layers of cell growth surfaces are capable of producing greater yields of adherent cells than commonly known flasks that permit growth of cells on a single bottom wall. While these multiple layer vessels allow for the growth of large numbers of cells, they present special challenges in day to day use.

There is a need for a cell culture vessel that can provide a device to direct fluid into and out of a cell culture vessel in a way that can be automated, and that improves the sterility of the transfer. In addition, there is a need for such a device that may be suitable for use in the performance of high throughput assay applications that commonly employ robotic manipulation.

### SUMMARY OF THE INVENTION

Embodiments of the present invention provide a multi-layer cell culture device having at least three cell culture chambers and at least two integral tracheal chambers, each cell culture chamber having at least one port, each port having a port cover where each port is structured and arranged to engage with a port cover to provide a releasable liquid tight seal. The port cover may be attached to the multi-layer cell culture device by a connector, which may be a hinged connector. The port cover has a septum. The septum may allow for a fluid flow device, introduced through the septum, to form a liquid-tight seal between the fluid flow device and the port. The fluid flow device may be a needle, a pipette or pipette tip, a tube or a cannula. In embodiments, the port may have a sealer which can be an annular structure to allow a port cover to connect to a complimentary structure on port to form a reversible liquid-tight seal.

The present invention provides a cell culture having a sliding port cover structured and arranged to engage with the ports to provide either an open or a closed port by slidingly engaging the port cover in an open position or a closed position in relation to the port. The sliding port cover can be connected to the cell culture device by a connector such as a hinged connector, or the sliding port cover can be integral with the cell culture device.

In the cell culture vessel having at least three rigid cell culture chambers, each cell culture chamber having at least one port; the at least one port may have protruding male feature structured and arranged to couple with a female fluid flow device. The cell culture vessel may have at least one port having a female feature structured and arranged to couple with a male structure of a fluid flow device. The cell culture vessel may have some ports with male structures and some ports with female structures to couple with complimentary structures of fluid flow devices.

This application describes a multi-layer cell culture device having at least three cell culture chambers and at least two integral tracheal chambers, each cell culture chamber having at least one port and a removable manifold structured and arranged to form a liquid-tight seal with the at least one port. The manifold has a valve. and can be structured and arranged to couple with at least one port of the one multi-layer cell culture device, or to couple one multi-layer cell culture device with a liquid reservoir.

This application describes a manifold having fluid flow devices structured and arranged to engage with the ports of more than one multi-layer cell culture device. Cell culture systems are also described which have at least one multi-layer cell culture device having at least two cell culture chambers, at least one external manifold having a manifold body and at least two fluid flow devices structured and arranged provide for the flow of fluid between the at least one external manifold and the at least two cell culture chambers of the multi-layer cell culture device; wherein fluid flows into the external manifold and is pooled in the manifold body before being distributed to the at least two fluid flow devices allowing fluid to flow between the at least one external manifold and the at least two cell culture chambers in parallel.

### BRIEF DESCRIPTION OF THE DRAWINGS

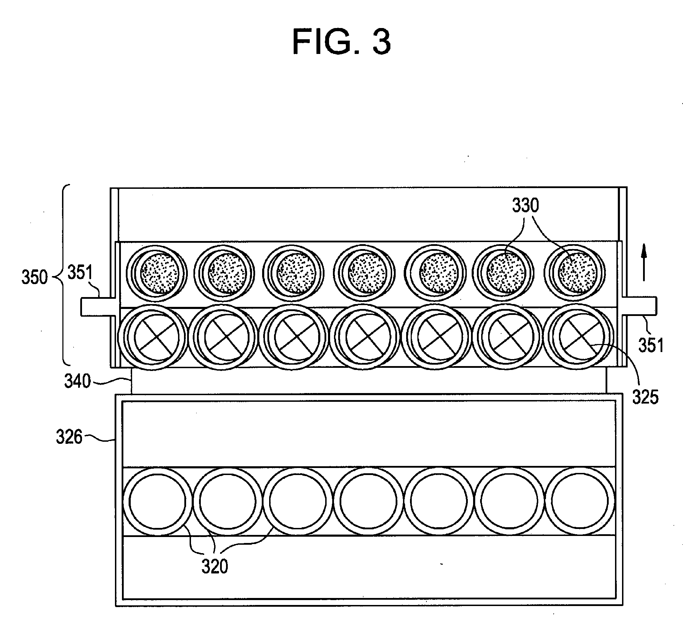

The invention is best understood from the following detailed description when read with the accompanying drawing figure 3.
Figure 1 is a partial cut-away perspective view of a multilayer cell culture device.
Figures 2A, 2B and 2C are illustrations of embodiments of ports and port covers of the prior art.
Figure 3 is an illustration of the port and sliding port cover of the present invention.
Figure 4 is an illustration of three connectors.
Figures 5A and 5B are illustrations of sealers.
Figure 6 is an illustration of a multi-layer cell culture flask illustrating ports and ports coupled to fluid flow devices.
Figure 7 is an illustration of ports and port couplings.
Figures 8A, 8B and 8C are illustrations of cannulas.
Figures 9A, 9B and 9C are illustrations of a manifold, showing valves.
Figure 10 is an illustration of a manifold showing coupling of the fluid flow devices to the manifold.
Figures 11A and 11B are illustrations of manifolds.
Figures 12A and 12B are illustrations of valves of the manifolds.
Figures 13A and 13B are additional illustrations of valves of the manifolds.
Figure 14 is an illustration of a manifold.
Figure 15 is an additional illustration of a manifold.
Figure 16 is an additional illustration of a manifold.
Figure 17 is an illustration of a multi-layer flask and its coupling with a manifold.
Figure 18 is an illustration of two multi-layer flasks and their coupling with a manifold.
Figure 19 is an illustration of a multi-layer flask and its coupling with a manifold.
Figure 20 is a perspective view of a manifold linked to a container.

### DETAILED DESCRIPTION

This application relates to a limited access cell culture system and device. The limited access cell culture device described has multiple layers of cell growth chambers in an integral multi-layer cell culture device, each layer having a port, reversibly sealable with a port cover, to allow for the introduction and removal of material into and out of the cell growth chamber. The device also can have an external manifold to control the flow of fluid into and out of the cell growth chamber.

Increasingly, cell cultures, particularly adherent cell cultures, are grown in stacked, space saving high density containers which minimize incubator space and maximize cell culture growth surface. See, for example, US Publication No. 2007/0026516. As cell culture containers become more and more efficient, and the spaces within them become more and more restricted, the practical use of these containers becomes complicated by the need to move small quantities of liquids into and out of these containers.

Maintaining the sterility of these high density containers and the fluid and cells contained within them is of the utmost importance. For example, vessels used to expand and treat cells in culture require that the cells be grown in a sterile system. One way to optimize the sterility of a cell culture container is to provide a closed or limited access cell culture system. A closed system may maintain the integrity of the cells in culture and prevent contamination. Cells in culture for therapeutic use may be unique to an individual, and may require conditions to promote proliferation and specific medical treatment without contamination. Cell culture vessels may have caps that are alternately removed and applied for access into the cell culture vessel, for example to add or remove cells or culture media. Or, alternatively, cell culture vessels may have a septum instead of cap. Culture contamination can result from material pushed into the vessel from the outside as the septum is punctured. The risk of contamination can be minimized by minimizing access to the cell culture chambers. For example, if a cell culture container is sterilized, and all of the interconnecting parts that come into contact with the cell culture container are sterilized, and manipulations of the cell culture container and the interconnecting parts is minimized and occurs in an aseptic environment, such as a hood or a laminar air flow enclosure, the risk of contamination is reduced. Additional materials and methods that decrease the risks of contamination will be discussed below.

Multi-layer cell culture containers must allow for entry and exit of cells and cell culture media into and out of the cell culture chambers. There is a need to facilitate the movement of fluids into and out of multi-layer cell culture containers in a way that maintains sterility and minimizes spills, while also minimizing the footprint of the multi-layer flask. Minimizing the footprint of these flasks and containers allows for increased utilization of space in incubators, as well as in storage and shipping. In addition, there is a need to provide these multi-level cell culture containers with features that support automated or robotic processes.

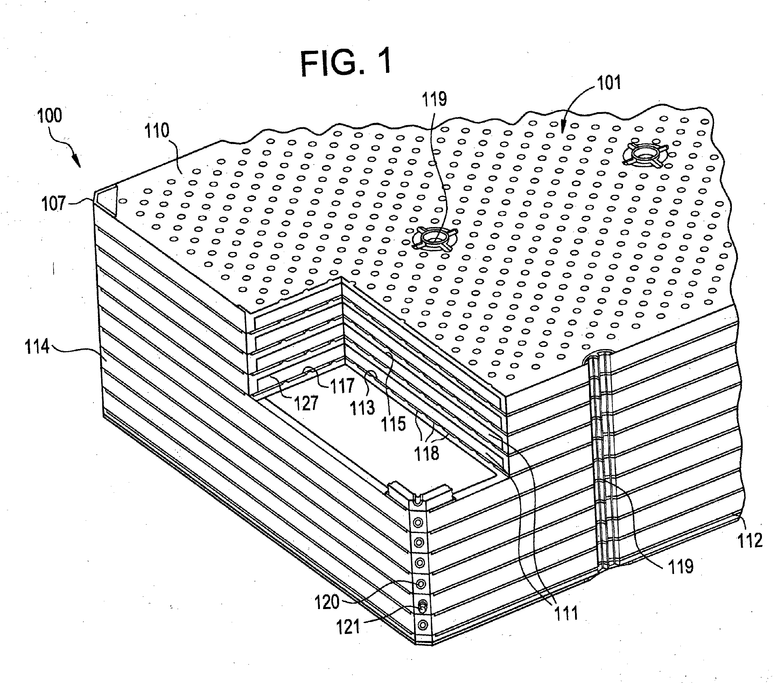

A multi-layer flask **100** is illustrated in the partial cut-away perspective view shown in **Figure 1****.** The multi-layer flask **100** has an outer vessel body **101** defined by a top plate **110,** a bottom tray (not shown), sidewalls **112,** and end walls **114.** Disposed within the flask **100** are individual cell growth chambers **111** as can be seen more clearly in the cut-away portion of **Figure 1****.** The individual cell growth chambers **111** are each defined by a bottom surface **113** and a top surface **115.** The surfaces **113** and **115** are attached to the flask body **101** along the sidewalls **112** and end walls **114.** Preferably, at least one bottom surface **113** within each chamber **111** is a gas permeable, liquid impermeable material capable of providing a surface for the growth of cells **117.** The gas permeable, liquid impermeable material may provide the surface upon which cells attach, or the floor of the cell growth chamber, or it may be the opposite surface, or the ceiling of the cell growth chamber. The bottom surface **113,** or the cell culture surface **113** may be flexible or rigid. Each top surface **115** is preferably a rigid, generally gas impermeable material that will provide support to the cell growth chamber **111.** The surfaces of the multi-layer flask may be clear, opaque, colored or colorless. In an embodiment of the present invention, there are tracheal spaces **118** between each cell growth chamber **111.** The opposing top surface **115** of the chamber **111** defines an upper wall to the cell growth chamber **111** as well as a bottom portion of a tracheal chamber **118.** The tracheal chamber **118** is therefore inclusive of a gas permeable, liquid impermeable surface **113** of a first cell growth chamber and an opposing surface **115** of a second growth chamber **111.** Supports **119** may also be present to provide structural support to integrally incorporate the surfaces **113** and **115** in forming growth chambers **111** in alternation with tracheal airspaces **118** within the unitary flask **101.** Each cell growth chamber **111** therefore alternates with a tracheal chamber **118** in vertical successive orientation.

The individual cell growth chambers **111** permit cellular growth on gas permeable membranes **113** such that multiple cell growth chambers **111** are integral with the body **101** of the multi-layer flask **100** and are capable of being completely filled with nutrient media for the growth of cells. The series of tracheal air spaces **118** through the multi-layer flask **100** provide gaseous communication between the cells **117** growing on gas permeable surfaces **113,** in media **127** in the individual cell growth chambers **111** inside the multi-layer flask, and the external environment. The tracheal spaces **118** allow oxygenation of media located within cell growth chambers **111** through the gas permeable surfaces 113. Further, the tracheal chambers **118** may take the form of any air gap or space, and do not allow entrance of liquid. As a result, a rigid cell culture multi-layer flask **100** having multiple growth chambers **111,** alternating with tracheal spaces **118,** is cooperatively constructed to afford the benefit of equivalent gaseous distribution to a large volume of cells **117.**

Gas permeable membrane **113** can be affixed to supports **119** and side walls **112** by any number of methods including but not limited to adhesive or solvent bonding, heat sealing or welding, compression, ultrasonic welding, laser welding and/or any other method commonly used for generating seals between parts. Laser welding around the circumference of the membrane **113** is preferred to establish a hermetic seal around the membrane region such that the membrane is flush with and fused to the face of the supports **119** such it becomes an integral portion of the interior surface of the multi-layer flask. Once the gas permeable membrane **113** is adhered to the sidewalls and endwalls, the top plate **110** and bottom tray **120** may be joined. The bottom tray **120** and top plate **110** may be injection molded. Various sizes and shapes of the supports **119** may be incorporated to facilitate positioning of the membranous layers **113** for cell culture **117** within the cell culture vessel **100.**

Gas permeable, liquid impermeable membranes **113** (see Figure 1) may be made of one or more membranes known in the art. Membranes typically are made of suitable materials that may include for example: polystyrene, polyethylene, polycarbonate, polyolefin, ethylene vinyl acetate, polypropylene, polysulfone, polytetrafluoroethylene (PTFE) or compatible fluoropolymer, a silicone rubber or copolymer, poly(styrene-butadienestyrene) or combinations of these materials. As manufacturing and compatibility for the growth of cells permits, various polymeric materials may be utilized. For its known competency, then, polystyrene may be a preferred material for the membrane (of about 0.003 inches in thickness, though various thicknesses are also permissive of cell growth). As such, the membrane may be of any thickness, preferably between about 25 and 250 microns, but ideally between approximately 25 and 125 microns.

The multi-layer flask **100** may be made by any number of acceptable manufacturing methods well known to those of skill in the art. In an embodiment of a method, the multi-layer flask **100** is assembled from a collection of separately injection molded parts. Although any polymer (such as polystyrene, polycarbonate, acrylic, polystyrene, or polyester) suitable for molding and commonly utilized in the manufacture of laboratory ware may be used, polystyrene is preferred. Although not required, for optical clarity, it is advantageous to maintain a thickness of no greater than 2 mm. The separate parts may be assembled by any number of methods including but not limited to: adhesive or solvent bonding, heat sealing or welding, compression, ultrasonic welding, laser welding and/or any other method commonly used for generating seals between parts such that it becomes an integral portion of the interior surface of the multi-layer flask. The top plate **110** and bottom tray may be aligned and joined, such as by laser welding.

Parts are held together and are adhesive bonded along the seam, ultrasonically welded, or laser welded, bonded using heat platens or by any other methods. Preferably, laser welding equipment is utilized in a partially or fully automated assembly system. The top plate and tray are properly aligned while a laser weld is made along the outer periphery of the joint.

Advantageously and in order to enhance cell attachment and growth, the surfaces internal to the multi-layer flask **100,** including the membrane layer, may be treated to enable cell growth. Treatment may be accomplished by any number of methods known in the art which include plasma discharge, corona discharge, gas plasma discharge, ion bombardment, ionizing radiation, and high intensity UV light.

An individual cell growth chamber may be bounded on one side by a layer of gas permeable membrane **110,** attached in a liquid impermeable manner to sidewalls **112** and on another side by a top surface that is a rigid layer, to provide a more rigid element to the individual cell culture growth chamber **111** and the multi-layered flask as a whole. For example, an individual cell growth chamber **111,** bounded on a top side by a rigid layer **115,** on its edges by sides, and on a bottom side by a gas permeable membrane. This individual cell growth chamber **111** can be stacked on top of another such individual cell growth chamber **111,** where the top portion of a rigid layer **115** of one individual cell growth chamber **111** forms a support structure that defines tracheal spaces underneath a gas permeable membrane **113** of the adjacent individual cell growth chamber. In an embodiment, individual cell culture chambers can be assembled into a larger multi-layer cell culture vessel. These individual layers can be snapped together, or otherwise attached to each other using any attachment method known in the art.

**Figure 1** illustrates alternating layers of tracheal air spaces **118** and individual cell growth chambers **111** which form the interior of flask **100.** The individual cell growth chambers **111** are defined by liquid impermeable, gas permeable membranes **113** attached in a liquid-impermeable manner to the sidewalls and endwalls of the cell culture vessel. Cell growth media **127** is contained between the membranes **113** and cells grow on the liquid-surface of these membranes **113.** In this embodiment, the cell growth chamber **111** may be formed by two layers of gas permeable membrane attached in a liquid impermeable manner to sidewalls **112** to form an individual cell growth chamber **111.** Tracheal air spaces **118** form layers between the gas permeable membranes, forming air pockets to allow the gas permeable membranes **113** to exchange air into the cell growth media **127.** Tracheal air spaces are supported by supports **119** which separate and support the layers of gas permeable membrane **113** which form individual cell growth chambers **111.** An advantage of a multi-layered flask compatible with a manifold is its enhanced capacity to grow cells on an opposing surface when the multi-layer flask is rotated 180°. Thus, when the multi-layer flask is rotated, cells can be cultured on an alternate gas permeable membrane surface **113.** Where only gas permeable membranes are layered intermediary to the multi-layer flask, cell growth is therefore enabled on both of its gas permeable surfaces **113.** The membrane **113** allows for the free exchange of gases between the individual cell culture growth chamber **111** and tracheal spaces **118.** Preferably a membrane **113** is included that is additionally durable for manufacture, handling, and manipulation of the multi-layer flask.

Accessibility to the cellular growth chambers **111** is achieved through ports **120** that extend through an external surface of a cellular growth chamber, to create an opening or a pass-through space in a surface of a cellular growth chamber **111.** While the port **120** is shown extending through a sidewall in Figure 1, the port **120** may be located on an endwall or on any other surface of the cellular growth chamber **111.** In embodiments of the present invention, each cellular growth chamber **111** has at least one port **120** allowing access into each cell culture chamber. More than one port **120** may be present in each cell culture chamber to allow fluid to enter a cell culture chamber **111** through one port **120** while displaced gas exits the cell culture chamber through another port **120.** A port cover **121** is shown in Figure 1. The port cover can be a plug. Port cover **121** may have a septum to prevent contamination of the contents of the cell culture chambers. Ports **120** may be treated with a layer of material to improve the water-tight capabilities of the ports. These materials may allow be more amenable to forming liquid-tight seals with cannula introduced into them, or with port covers. Examples of these materials include rubber, PVC, Teflon®, cork, silicone, gum, urethane, or any other material known in the art.

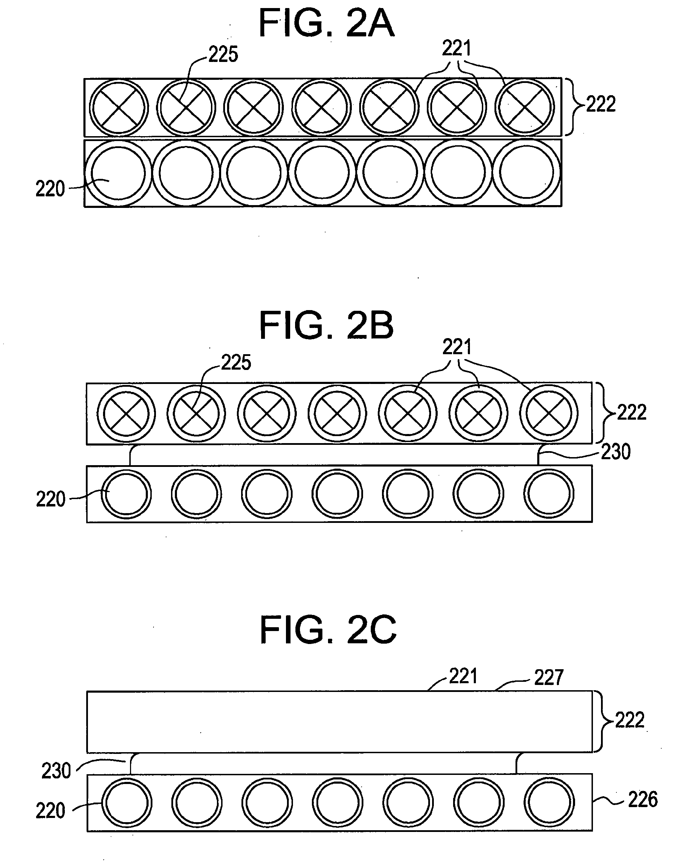

**Figure 1** illustrates a multi-layer cell culture device having a set of aligned ports **120,** on a corner **107** of the multi-layer flask **100.** The corner **107** may be flat, as shown in Figure 1, or rounded, or any shape. **Figure 1** also illustrates port cover **121** which provide a releasable closing on the port **120.** Port cover **121** may be individual port covers **121,** as shown in **Figure 1****,** or may be in strips, as shown in **Figure 2****,** structured and arranged to close a strip of ports.

**Figures 2A, 2B** and **2C** illustrate embodiments of ports and port covers not according to the present invention of the present invention. As shown in Figure 2A-2C, ports **220** may be structured and arranged to reversibly engage with port covers **221** to form a water-tight seal. Port **220** and port cover **221** may form a water-tight seal by forming a friction seal between the parts. Port covers **221** may be in strips 222 structured and arranged to form liquid-tight seals against a plurality of ports **220.** Ports **220** may be openings that are flush with the surface of the multi-layer flask or may be raised structures, extending from the surface of the multi-layer flask, defining an opening. These raised structures may be protruding structures that provide a male coupling structure to enable the port to couple with a female port cover or a female fluid flow device. Or, the port may be a female structure to provide coupling structure for a male port cover or a male fluid flow device.

As illustrated in Figure 2A, port covers **221** may fit within ports **220** to reversibly engage with ports **220** to form liquid-tight seals. Or, as illustrated in Figure 2B, ports **220** may fit within port covers **221** to reversibly engage to form liquid-tight seals. As illustrated in Figures 2A and 2B, port cover **221** may contain septa **225.** Port **220** contains septa **225** (not shown).

As illustrated in Figure 2C, ports may be surrounded by a port wall **226,** a raised portion surrounding the ports. The port cover **227** may reversibly engage with port wall **226** to form a liquid-tight seal. Port cover **227,** made from flexible plastic, may fit snugly inside port wall **226,** or may fit snugly to form a friction fitting outside port wall **226,** to form a reversible liquid-tight seal. In embodiments, the port cover **221** may be attached to the multi-layer flask by a connector **230.**

In Figures 2A-2C, ports **220** may be open or sealed by a port cover **221** or **226.** Or, ports **220** may be open, and may contain a septum **225.** Multi-layer flasks **100** of the present invention may be manufactured, sterilized, packaged into sterile packaging and transported with open ports **220.** When they are ready to be used, a multi-layer flask **100** may be placed into an aseptic environment, such as a hood or laminar air flow enclosure, and its sterile packaging may be opened. Liquid may be introduced into the multi-layer flask using a fluid flow device, a device for directing fluid from one place to another, which may include needles, cannula or pipettes, pipette tips, tubes, lines, channels, pipes, ducts, conduits, or any other fluid flow devices known in the art. Once the flask **100** is filled, a port cover **221** or **226** may be placed over the open ports **220** to seal them, before the sealed multi-layer flask is introduced into an experimental environment, such as an incubator. The port cover **221** or **226** may be provided in separate sterile packaging, or may be included in the sterile packaging that contained the multi-layer flask.

Or, in an alternative embodiment, the multi-layer flask of the present invention may be manufactured and sterilized, and the ports may be sealed with a sterile port cover **221** or **226.** The sealed sterile multi-layer flask may then be packaged and transported. When ready to be used, the sealed flask may be placed into an aseptic environment such as a hood or laminar flow enclosure, removed from its packaging, and liquid may be introduced into the flask either by opening the ports by removing the port covers, or by introducing liquid into the flask by inserting a needle, cannula, pipette tip, or other device through septa **225** within the port cover **221,** and introducing liquid into the multi-layer flask.

**Figure 3** illustrates a port and port cover of the present invention. Figure 3 illustrates ports **320** and a sliding port cover **350.** The sliding port cover **350** engages with the ports **320,** or a port wall **326,** to form a releasable water-tight seal. In this embodiment, the sliding port cover **350** has a sliding mechanism to allow the user to choose the desired port cover by sliding the handle **351,** as indicated by the arrow. As illustrated in Figure 3, the sliding port cover **350** allows the user to choose whether the ports will be covered by open or closed, or covered by septa **325,** or filters **330.** Filters **330** may be filter materials known in the art to reduce or prevent contamination of cells in culture. The sliding port cover may be set in an open position, allowing for the free flow of air into and out of the cell culture chamber. In this open position, the port is covered with a filter to allow / for the flow of air into and out of the cell culture chamber, but prevent contamination. Or, the sliding port cover may be set in a closed position, where the closed position has a septum, to allow a fluid flow device such as a needle or a pipette tip to enter the cell culture chamber through the closed port cover. For example, the sliding port cover **350** is set to cover the ports **320** with septa **325** by sliding the sliding port cover in the direction of the arrow shown in Figure 3, until septa **325** are aligned with ports **320.** A user may introduce liquid through the ports **320** by inserting a needle or cannula or pipette tip through the septa **325.** The septa **325** may be structured or arranged to accommodate a liquid-handling device such as a small or large bore needle, a cannula, or a pipette tip. Once liquid has been introduced through ports **320,** the user may choose to leave the ports covered with septa **325,** or the user may choose to cover the ports **320** with filters. If the user chooses to cover the ports **320** with filters **330,** the user would slide the slide handle in the opposite direction of the handle, to align the filters **330** with the ports **320.** In this way, the user may change these coverings by sliding the handle of the port cover from one position to the other. In this embodiment, the sliding port cover **350** may be integral with the multi-layer flask of the present invention, or the sliding port cover may attached to the multi-layer flask by a connector **340.** The sliding port cover may be removable. If the sliding port cover **350** is removable, it may be attached to the multi-layer flask by any connector **340** such as a hinged connector **340.**

When a port cover contains a septum, and the port cover is engaged with the port to form a reversible liquid-tight seal, the septum may be situated above the port. That is, when the port cover contains a septum, the septum itself may be seated on top of or above the port.

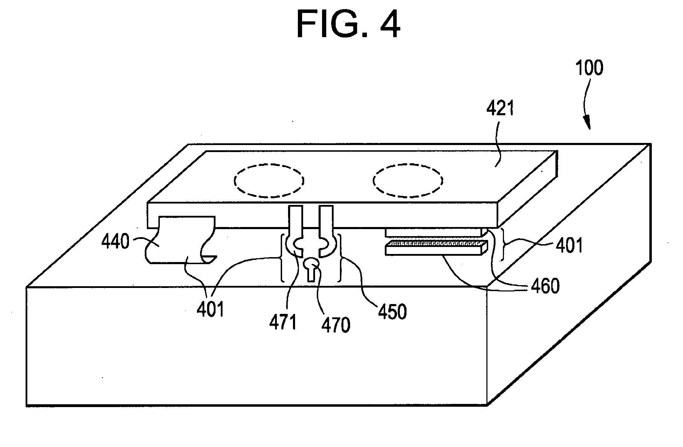

Turning now to **Figure 4,** Figure 4 illustrates connectors **401**. Connector **401** is a feature which connects the port cover **421** to the multi-layer flask. The connector **401** may be a hinged connector **440.** A hinged connector may be a flexible thin ribbon of plastic that is attached on one side of the ribbon to the port cover and on the other side of the ribbon to the port wall or to the multi-layer flask. This thin flexible ribbon of plastic is deformable, therefore allowing port cover to be attached to the multi layer flask either in an open position (not shown), or in a closed position, as shown in Figure 4. The hinged connector **440** may be a thicker ribbon of plastic that has been scored to allow the plastic to bend at the score marks. Or, the hinged connector may be a pivot hinge, a spring hinge (to ensure that the port cover is in a closed position or in an open position) or any other type of hinge known in the art. These hinged **440** connectors may be molded separately, or molded as a part of the port cover, and attached to the multi-layer flask by any method known in the art, including the methods for molding and attaching plastic parts as discussed above.

The connector may be a ball and socket connector **450.** A ball feature **470** may protrude from the port cover or the multi-layer flask, and may reversibly engage with protruding socket features **471** in the opposite surface. The port cover may then be snapped into the ball and socket joint to connect the two pieces. In additional embodiments, the connector may be a hook and loop fastener **460,** zip-lock type fastener, adhesive, or other connecting mechanisms.

The port cover **421** may be connected to the multi-layer flask **100** (as shown in Figure 4), or to a structure of the multi-layer flask **100,** such as a port wall **226** (as shown in Figures 2A-2C) by a connector **401.** Or, alternatively, port cover **221** or port cover strip **222** may not be attached to the multi-layer flask but may be separate from the multi-layer flask 100. Port cover **221** or port cover strip **222** may be disposable, so that each time the cell growth chambers are accessed through ports **220,** the used port cover **221** or port cover strip **222** is removed and discarded, and a new port cover **221** or port cover strip **222** is applied to close the ports **220** when the user wishes to close the multi-layer flask. Port cover or port cover strip may be disposable and sterilizable by heat sterilization or UV sterilization, or any other method known in the art.

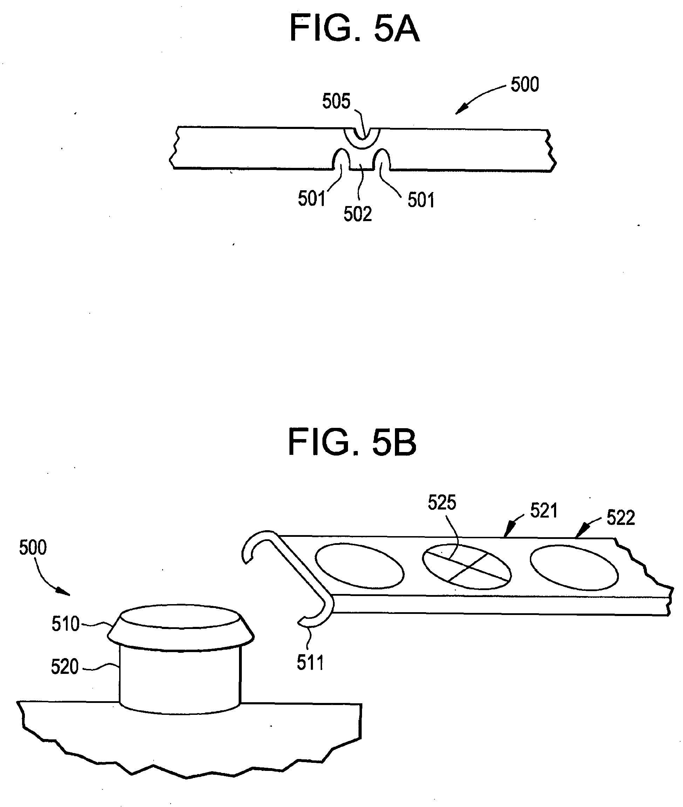

Ports **220** and port covers **221** or **226,** may have internal or external sealing or engaging structures to allow the port covers to engage against the ports to provide a liquid-tight seal. See for example, **Figure 5A** and **5B.** Figure 5A illustrates a zip-lock type sealer **500.** A pair of spaced, parallel fastener ridges **501** forming a channel **502** may be provided on one surface, for example the top surface of a port or a port cover, or a port wall, and the complimentary surface may have a single ribbon of flexible material **505** that, when pressed into the fastener channel, forms a releasable liquid-tight seal. The fastener may be designed to change color when the seal is formed. For example, the fastener ridges may carry a color, for example blue, and the ribbon may carry another color, for example red, and wen the two complimentary elements are pressed together to form a seal, a purple color may show through the structure.

**Figure 5B** illustrates a sealer **500.** A ledge structure **510** or annular structure around the raised port **520** engages with complimentary structures which may be, for example, a flexible catch bar **511** on the port cover **521** or port cover strip **522** to allow the port cover **521** to slidingly engage with the ports to create a releasable liquid-tight seal between the port cover strip **522** and the port(s) **520.** While only one port **520** is shown in Figure 5B, the port cover strip can be used to slidingly engage with a series of similarly structured ports to form seals between a series of ports **520** and a port cover strip **522.** These structures taken together, the ledge structure **510,** which slidingly engages with the port cover strip **522** to form a liquid-tight seal, are an embodiment of a sealer **500.** Or, in additional embodiments, port cover **521** may have internal or external sealers, structures to allow the port covers **521** to engage against the ports **520** to provide a liquid-tight seal. As shown in Figure 5, port cover **521** may slide onto port **520** to fit on top of port **520** to form a liquid-tight seal. This places a septum **525,** contained in the port cover **521,** above the port **520,** but engaged with the port to form a liquid-tight seal. Port **520** or port cover **521** may have a septum **525.** If the septum **525** is in the port cover **521,** the septum **525** does not extend down into the port, but is above the port and outside the port. The septum **525** allows the port to be closed unless a needle or cannula or other device is inserted through the septum **525** into the port or port cover. When a needle or cannula or other device is inserted through the septum **525** into the port **520** or port cover **521,** the needle or cannula can pass through the port or port cover to allow access into the cell culture chamber of the multi-layer flask, while maintaining a liquid-tight seal between the port or port cover and the needle or cannula.

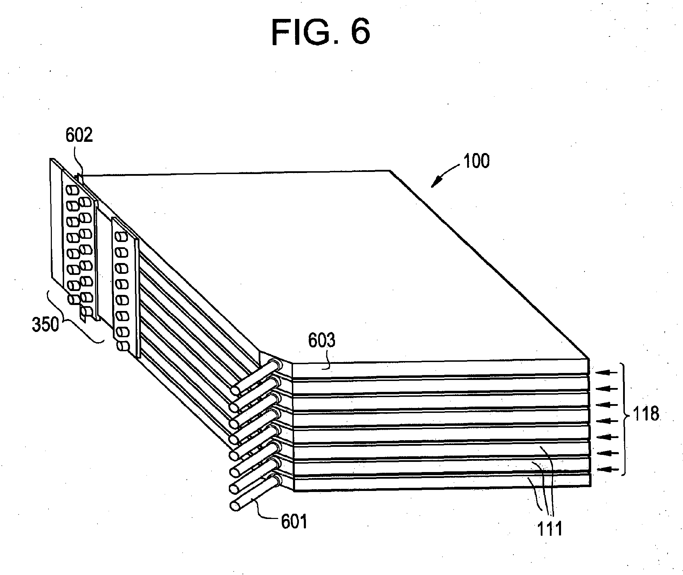

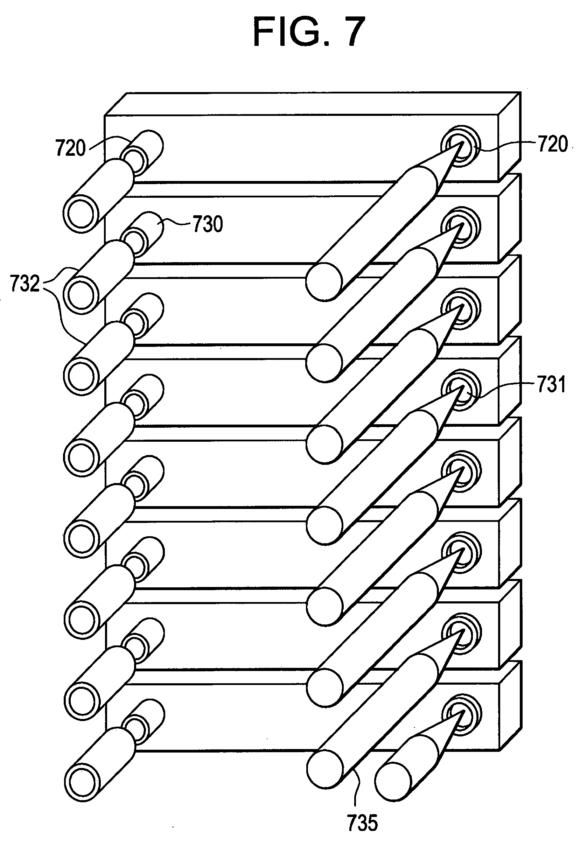

Turning now to **Figure 6, Figure 6** illustrates a multi-layer flask **100** incorporating the sliding port cover **350** shown in Figure 3 on a first end **602** of the multi-layer flask **100,** and tubes **601** engaged with ports (not shown) on a second **end 603** of the multi-layer flask **100.** The multi-layer flask has multiple cell growth chambers **111** and integral tracheal air spaces **118.** Each cell growth chamber **111** can be accessed through a port (see Figure 7). In an embodiment of the present invention, a multi-layer flask **100** can be filled with fluid by pumping fluid into each cell growth chamber **111,** through a tube **601.** For example, filters may be placed over the ports on the first end **602** of the multi-layer flask **100,** by sliding the sliding port cover to the filter position, as shown in Figure 3. Fluid can then be pumped into each cell growth chamber **111** through a tube **601.** Air or other gas, displaced by the fluid entering the cell growth chambers **111** through the port on the second end **603** of the multi-layer flask **100,** can exit the cell growth chambers through the filtered port on the sliding port cover **350.** Once the multi-layer flask is filled, the sliding port cover **350** may be adjusted to cover the ports on the first end **602** of the multi-layer flask with septa to form a liquid-tight seal. Tubes **601** may be sealed by clamping or by welding the tubes closed. The sealed multi-layer flask may then be placed into an appropriate environment, an incubator for example, to allow cells to grow in the multi-layer flask. Fluid may be pumped into a set of cell growth chambers **111** through a first set of tubes **601,** may flow through cell growth chambers **111,** and may exit cell growth chambers through a second set of ports, coupled to a second set of tubes or other fluid flow devices.

Tubes **601,** attached to the multi-layer flask may allow for more sterile transfer of fluid into and out of the multi-layer flask. Tubes can be coupled to additional tubes or other fluid-flow devices using couplers such as male or female structures which accommodate tubes in friction connections to connect a tube to another tube or another structure. Tubes can be heat-welded together to form uninterrupted sterile fluid flow devices. For example, fluid flowing through heat-welded tubes may connect a multiwell flask to a source or sink of fluid that may be a distance away from the multi-layer flask. To remove or interrupt the connection, tubes need only be cut or folded and clamped or heat welded closed. The multi-layer flask illustrated in Figure 6 may be assembled to include tubes, sterilized, packaged and shipped so that a user may open sterile packaging, heat-weld the tubing to connect the multi-layer flask to a sink or source of fluid, and use the flask. This flask configuration may decrease the risk of contamination by removing potentially contaminating liquid-handling features such as valves and couplers.

**Figure 7** represents an expanded view of the port area and illustrates connections between ports and tubes or cannula in embodiments of the present invention. Ports **720** can have male **730** or female **731** structures. Male ports **730** can have protruding structures which allow needles, pipette tips, tubes, **732** cannula, or other fluid-flow devices to fit snugly around the male port **730** to form a liquid-tight seal. Female ports **731** can have receptacle structure to allow cannula **735** needles, tubes or pipette tips or other fluid flow devices to fit snugly into the female port **731** to form a liquid-tight seal.

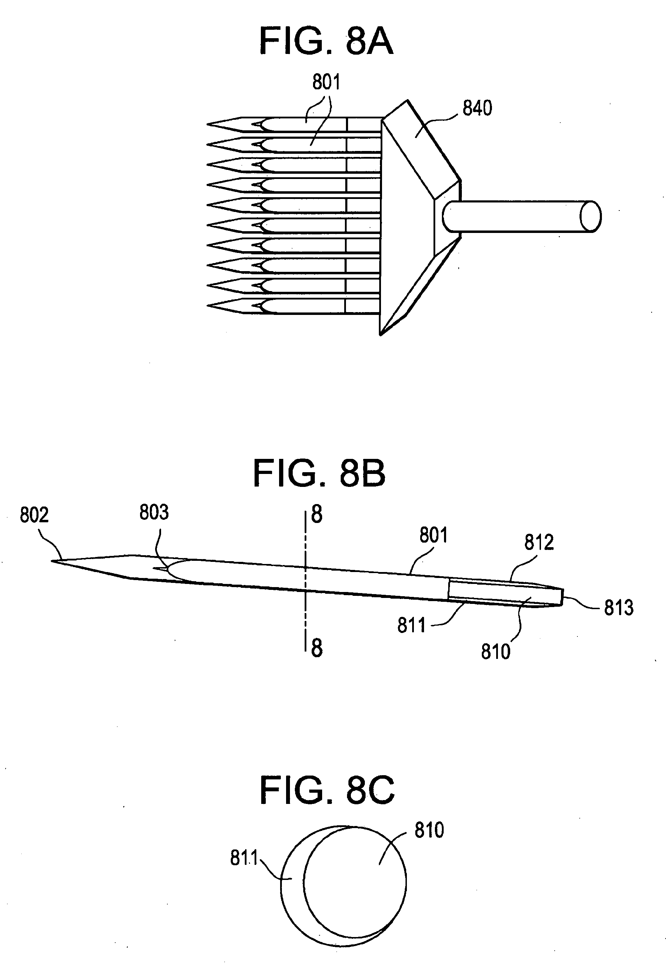

Fluid flow devices, include needles, cannula, pipette tips or tubes and are devices which allow fluid to be directed into and out of a multi-layer flask. **Figures 8A-8C** illustrate one such fluid flow device, a cannula **801.** Figure 8A illustrates multiple cannula **801,** attached to a manifold **840.** The cannula may be attached to the manifold at their proximal ends **813.** In an embodiment, a cannula **801** of the present invention may allow for the directional flow of liquid, while also allowing for a separate flow, or venting of air or gas. For example, the cannula shown in figure 8 has a sharp distal tip **802** for piercing a septum and a second tip **803** which is proximal to the first tip. The second tip **803** may also be sharp, and is associated with a second flow path **811.** When examined in cross-section as seen in Figure 8C, which is a cross-sectional illustration, taken at the line 8-8 shown in Figure 8B, the cannula has two separate flow paths, one for liquid **810** and one for air **811.** When the distal end of the cannula **802** is inserted into a port of a multi-layer flask as shown in Figure 7, liquid can flow into the multi-layer flask through the liquid path **810,** and displaced air can escape from the multi-layer flask through the same cannula through the air path **811,** which vents to the outside air through air vent **812** which may be located at the proximal end **813** of the cannula. This air vent may be covered with filter material to prevent contamination from this air path. Using this cannula embodiment, liquid can be introduced into a closed cell culture chamber, and displaced air can be vented out at the same time, without the need for a second open port in each cell culture chamber to allow for the release of displaced air. The cannula can be a rigid tubular structure defining a first interior path structured and arranged to conduct fluid and a second interior path structured and arranged to conduct fluid and having a proximal and a distal end. These fluid flow devices may be coupled to a manifold. A manifold is a device structured and arranged to hold multiple fluid flow devices. The manifold may also be structured and arranged to direct fluid into and out of multiple fluid flow devices. For example, the manifold may be structured and arranged to hold multiple cannula or tubes or pipette tips that are spaced in a way to ensure that the multiple fluid flow devices align with ports of a multi-layer cell culture structure. The manifold may be internal (integral with the multi-layer cell culture structure) or external (separate from the multi-layer cell culture structure).

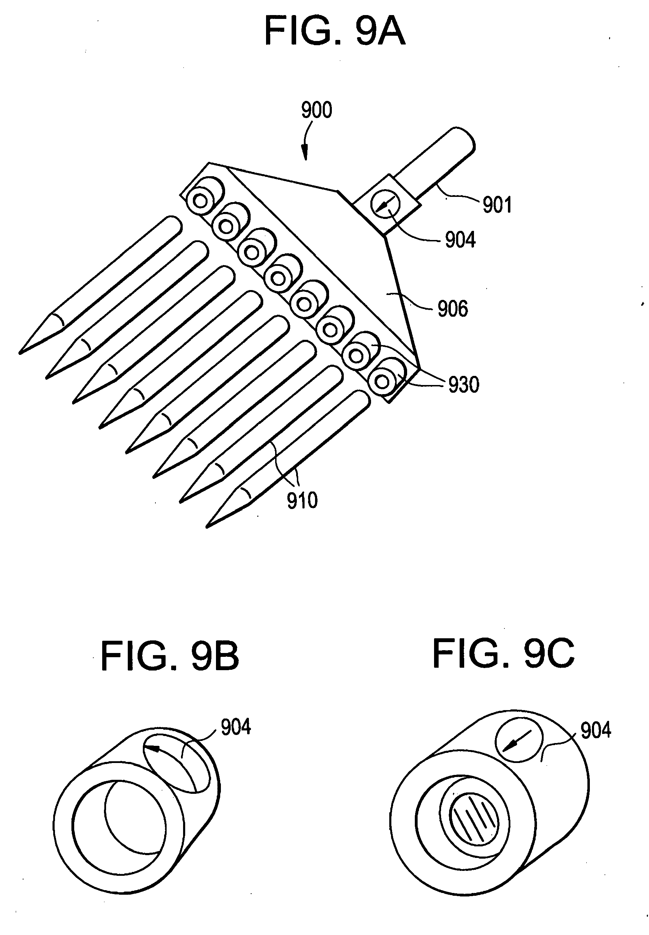

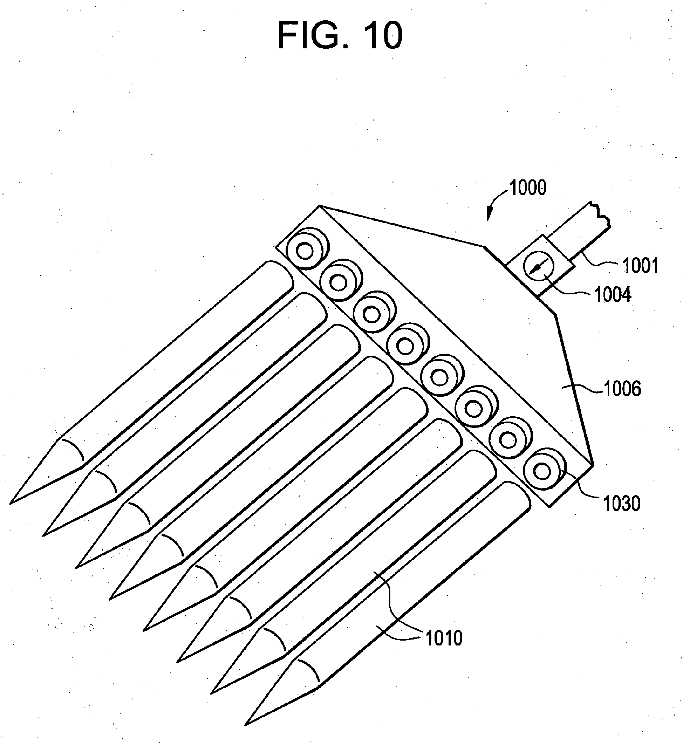

**Figure 9A** illustrates an external manifold **900**. The manifold **900** is a device for the manipulation of flask contents as they enter and exit the multi-layer flask. The external manifold **900** has couplers **930** to couple fluid flow devices or cannula **910** to the manifold **900.** These couplers **930** may be female structures (as shown), and fluid flow devices such as pipette tips or cannula may insert into these female structures to couple the fluid flow devices to the manifold. Or, the couplers **930** may be male couplers as shown in Figure 10.

**Figure 10** illustrates an additional external manifold **1000**. The manifold **1000** shown in Figure 10 has a necked opening **1001,** a valve **1004** interposed between the necked opening and the manifold body **1006,** male couplers **1030** structured and arranged to couple with fluid flow devices **1010** such as cannula, pipette tips or tubing.

Fluid flow devices such as pipette tips or cannula may be structured and arranged to insert into the cell growth chambers through the ports **120** of the multi-layer flask **100.** For example, fluids entering the manifold through the necked opening **1001** of the manifold may flow into the manifold body **1006,** and through the cannula **1010.** When these cannula **1010** are inserted into the ports **120** of the multi-layer flask **100** (see Figure 1), fluid can flow through the cannula **1010** into the interior of individual cell culture chambers.

Referring again to Figure 9, the external manifold **900** may have a valve **904.** In an open position, as shown in Figure 9B, fluid may flow freely through the valve **904** from the necked opening **901** to the manifold body **906.** In a closed position, as shown in Figure 9C, fluid may not pass from the necked opening **901** to the manifold body **906.** This valve may be operable by rotating a valve key, accessible from the exterior of the external manifold **900.** The external manifold illustrated in Figure 9 may connect multiple cell culture chambers in a multi-layer cell culture flask in parallel. That is, fluid entering the manifold body **906** through the necked opening **901** may flow through multiple fluid flow devices **901** to enter multiple cell culture chambers at the same time. External manifolds of the present invention may be structured and arranged to allow for fluid to flow from one cell culture chamber in one multi-layer cell culture device to another cell culture chamber in another multi-layer device through the manifold without mixing with fluid bound for another cell culture chamber. Fluid passes from one multi-layer cell culture device to another in series.

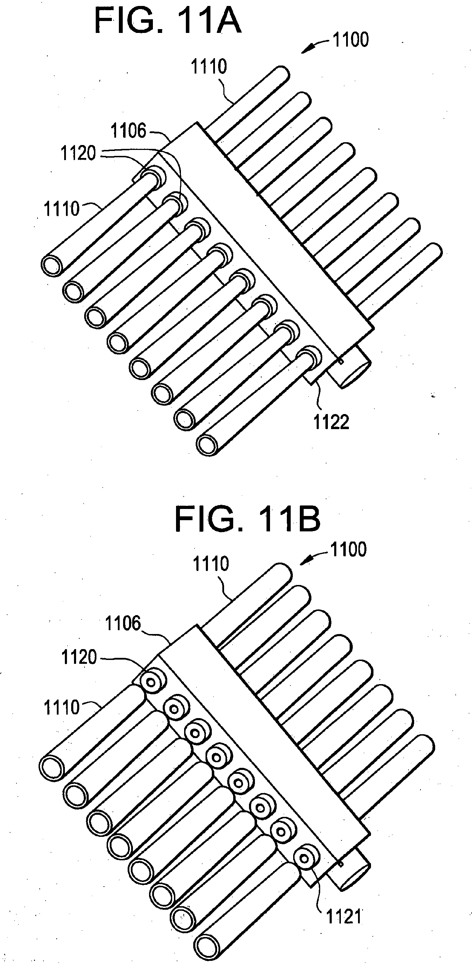

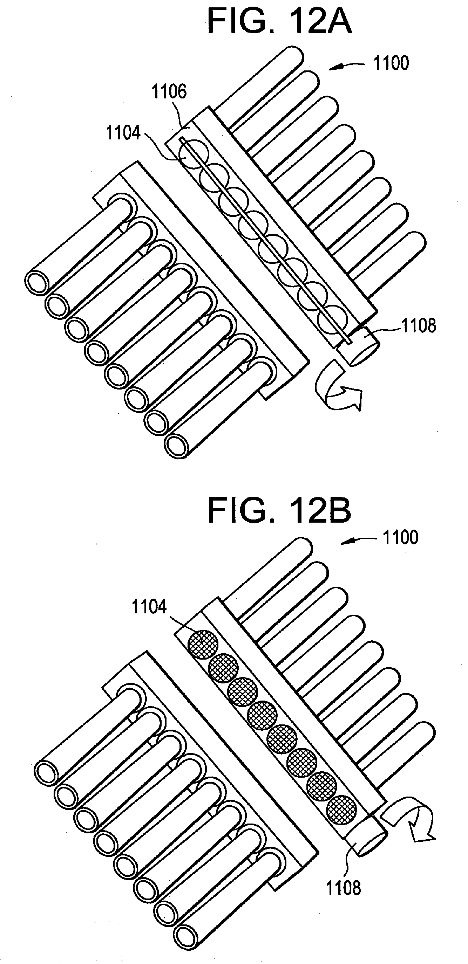

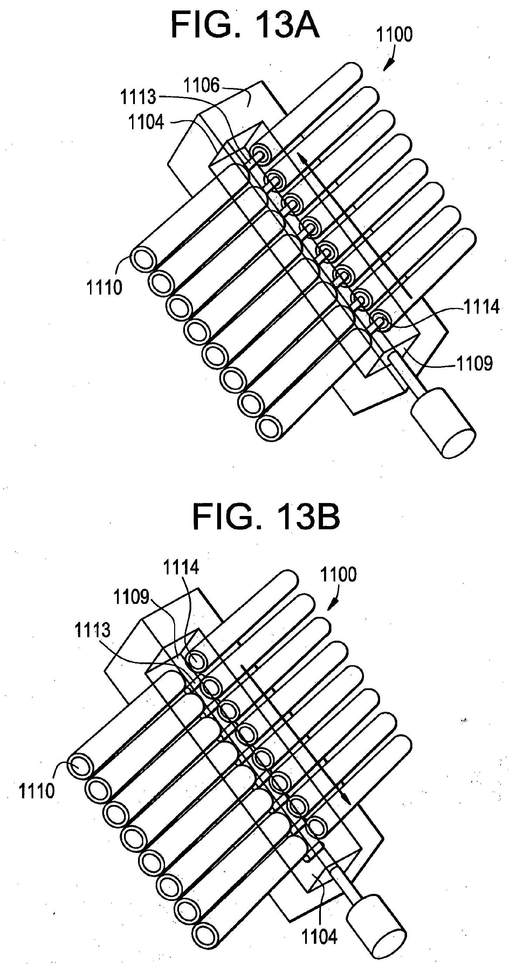

**Figure 11A** and **11B** illustrate a series manifold **1100.** Fluid flow devices 1110, in this case tubes, are attached to a manifold body **1106.** The manifold body **1106** has ports **1120** which are either male **1121** or female **1122** and are structured and arranged to form liquid-tight seals with the tubes **1110.** The series manifold **1100** may have a valve to allow fluid to flow from one side of the series manifold to the other side of the series manifold, or to stop fluid from flowing through the manifold body **1106.** **Figures 12A** and **12B** illustrate a valve **1104** for the series manifold **1100** shown in Figure 11A and 11B. In this embodiment, the valve may operate to open (as shown in Figure 12A) or close (as shown in Figure 12B) a passageway through the manifold body **1106** from one side of the manifold to the other. A valve opening device **1108** operates to switch the valve between the open and closed position. As shown in Figure 12A and 12B, the valve opening device may rotate to open or close the valve as in a butterfly valve. In an alternative embodiment, as illustrated in **Figures 13A** and **13B****,** the valve opening device **1109** may operate by sliding the valve opening device **1109** from an open position to a closed position, as shown by the arrows. When the valve is open, a passageway **1113** is aligned with openings **1114** on each side of the manifold **1100.** When the vale is closed, the passageways **1113** are not aligned with the openings **1114** and no fluid can flow from one side of the manifold to the other. While three embodiments of valves have been particularly illustrated, many valve mechanisms are known in the art and may be applicable in embodiments of the present invention. Valves may be mechanical or electronic, for example solenoid valves or magnetic valves may be used.

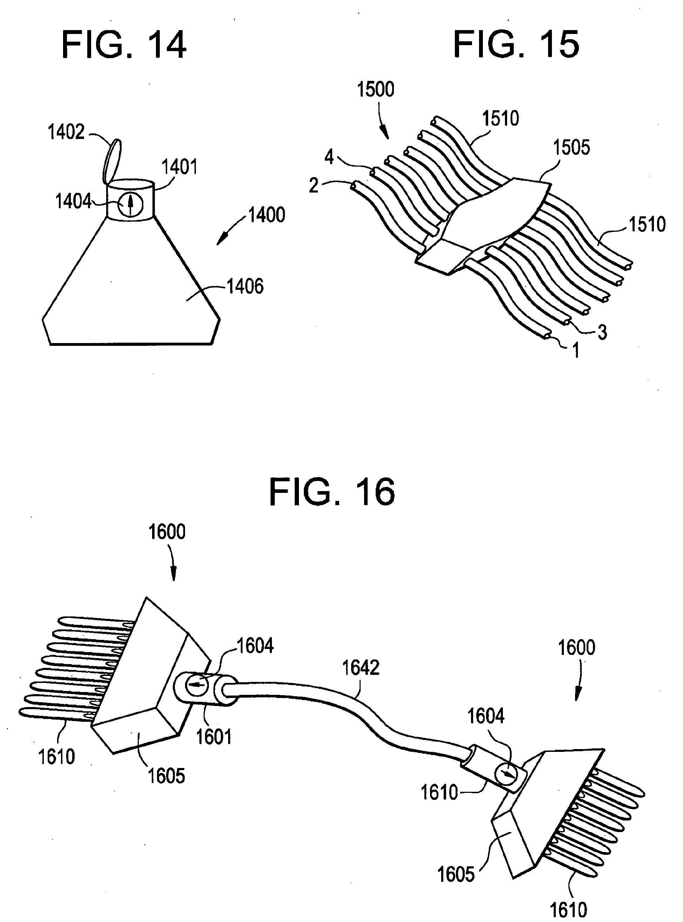

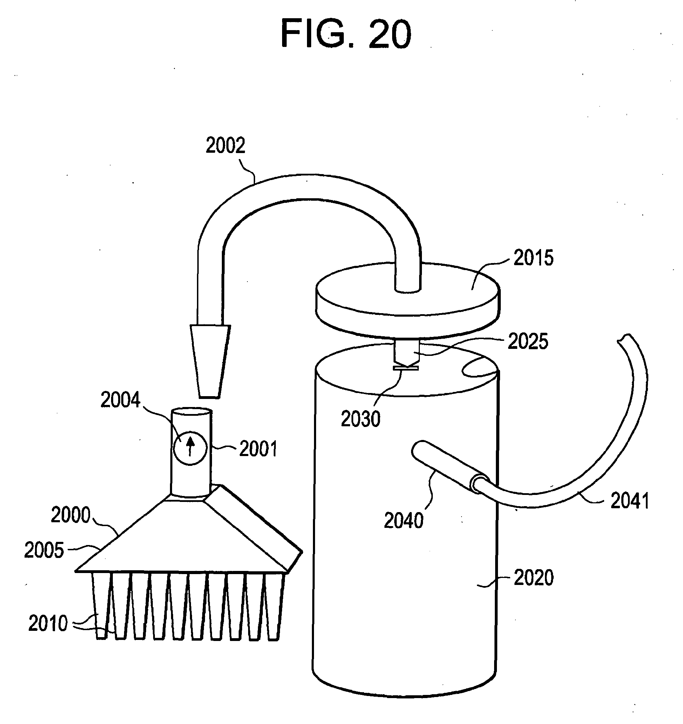

External manifolds are illustrated in Figures 14 - 16. **Figure 14** illustrates an external manifold **1400** having a necked opening **1401,** a manifold body **1406** and a valve **1404.** The necked opening can be covered by a cap **1402.** The cap **1402** may be present or absent. If present, the cap may incorporate filters to allow for the exchange of gas between the internal and external spaces of the cell culture system. The necked opening can be attached to tubing which allows for fluid communication from a reservoir of fluid to the manifold, to the multi-layer flask (see Figure 20).

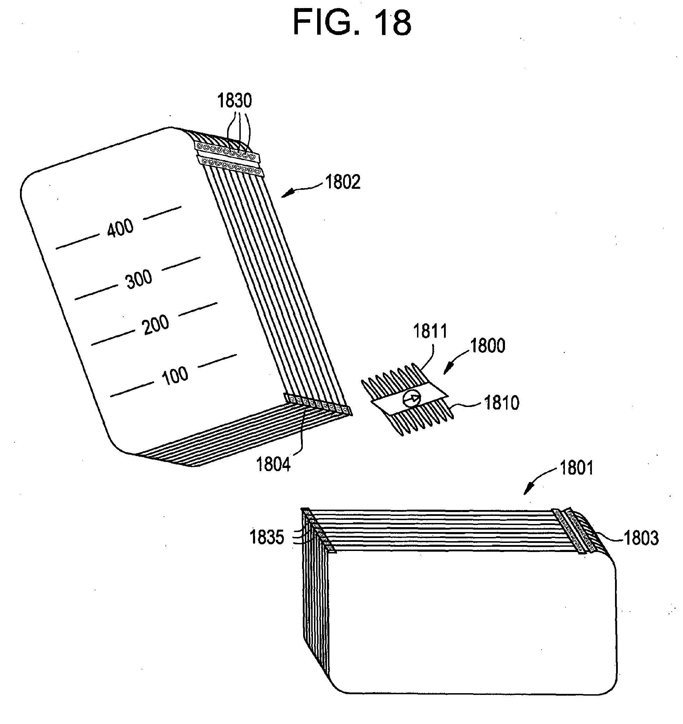

**Figure 15** is an illustration of an additional manifold **1500**. The manifold body **1505** connects two sets of tubes **1510,** structured and arranged to insert into two adjacent multi-layer flasks, as shown in **Figure 18****.** It is possible to connect adjacent multi-layer to form a network of multiple multi-layer flasks. In addition, if this manifold embodiment is used, it is possible to fill multiple multi-layer flasks with fluid by administering fluid to one multi-layer flask. For example, two or more multi-layer flasks can be connected together using this manifold embodiment **1500.** Fluid can be administered to a multi-layer flask on top of a stack of connected multi-layer flasks. To fill all of the flasks, fluid is allowed to flow from the top flask, through all of the intermediary flasks to a bottom flask until all of the flasks are filled with fluid. Depending upon the internal structure of the manifold, whether fluid is pools in the manifold body **1505,** or whether fluid flows from a tube on one side **1510** of the manifold body to a corresponding tube on the other side of the manifold body **1510,** fluid can flow through this embodiment of the manifold to multi-layer cell culture flasks in series or in parallel. For example, if the manifold body **1505** allows fluid to mix as it enters the manifold from the cannula, this manifold allows all of the layers within a single multi-layer flask, or between multi-layer flasks to mix, and thereby be connected in parallel. If the manifold body maintains defined connections, it can connect cell culture chambers from flask to flask in series. For example, if tube 1 is connected only to tube 2 through the manifold body, and if tube 3 is connected only to tube 4 as it passes through the manifold body, then the cell culture chamber that is accessed by tube 1 is connected in series with the cell culture chamber that is accessed by tube 2, and the cell culture chamber that is accessed by tube 3 is connected in series with the cell culture chamber that is accessed by tube 4.

Tubes **1510** may be replaced by cannula or needles or other fluid flow devices structured and arranged to connect in a liquid-tight manner to the manifold and to the multiple-layer flask. For example, tubing can fit in a friction fit over a port when the port has a height and extends above the surface of the multi-layer flask. Or, in the alternative, tubing may be inserted into a port to form a liquid-tight seal when the port is structured and arranged to form a liquid-tight friction fit between the tubing and the port.

Figure 16 shows two manifolds **1600** each having a manifold body **1605** connected to cannula **1610,** a valve **1604,** and a necked opening **1601** connected to tubing **1642** which connects the two manifolds together. It will be understood by those of skill in the art that the manifolds of the present invention may exist in any configuration and may connect one multiple layer flask with another multiple layer flask, may connect a multiple layer flask with a liquid reservoir or a waste container, may connect one manifold with another manifold, and may provide multiple connections through known connectors, valves, pumps or other connections.

In all manifolds the distance between the cannula can correspond to the distance between the ports of cell culture chambers in the multi-layer flask. The number of cannula present can correspond to the number of layers or cell culture chambers in the multi-layer flask. Manifolds may be packaged in-place in a multi-layer flask, to be removed by the user, or may be packaged separately from the multi-layer flask. Manifolds, multi-layer flasks, port covers, port cover strips, tubing and all of the features and accessories described herein may be sterilized and sold in sterile packaging, together or separately.

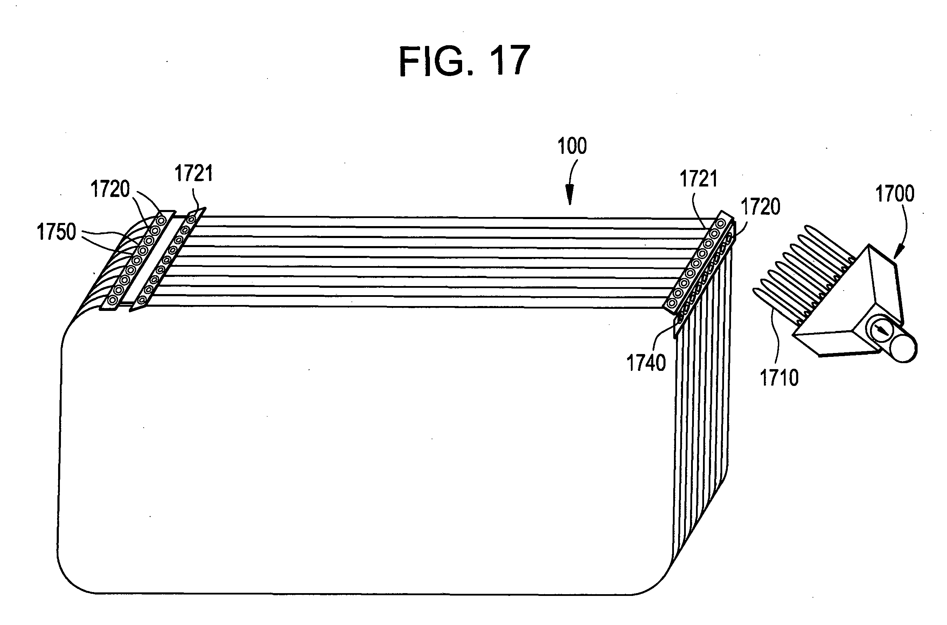

Turning now to **Figure 17****,** there may be ports **1720** on two sides of the multi-layer flask **100,** which may be entry ports **1740** and exit ports **1750.** In an embodiment, as fluid enters the multi-layer flask **100** through cannula **1710** of a manifold **1700** inserted into the entry ports **1740,** displaced fluid or gas can leave the multi-layer flask **100** through the exit ports **1750** on the other side of the flask. Fluid can be moved through the manifold by providing positive or negative pressure to the manifold, for example by using a pump or a vacuum attached to the manifold or the flask (see Figure 20). Once the cell culture chambers of the multi-layer flask **100** have been completely filled with fluid, the cannula **1710** of the manifold **1700** can be removed from the entry **ports 1740,** and both the entry ports **1740 and** the exit ports **1750** can be plugged with port covers **1721.** The multi-layer flask **100,** in this configuration, with both entry and exit ports closed by port covers, is a closed multi-layer cell culture system. This closed multi-layer flask can then be rotated to maximize the cell growth surface within the cell growth chambers, (i.e., to put the bottom plate down) and placed in an appropriate location for cell culture, such as an incubator. In additional embodiments, the manifold **1700** may not have cannula but may form a liquid-tight seal directly with ports (see for example, Figure 10).

When the time comes to empty the cell culture chamber, the multi-layer flask **100** can be removed from its location for cell growth, rotated so that the ports are in an "up" position, the port covers **1721** on both the entry and the exit ports can be removed, either manually or by robotic manipulation, and the cannula of a manifold can be inserted into the multi-layer flask to remove fluid, either by suction or by gravity. As the multi-layer flask empties, the flask can be tilted so that the remaining fluid is presented to the tips of the cannula, extending into the multi-layer flask **100.**

**Figure 18** is a perspective view showing how multiple flasks can be connected together by a manifold. When the cannula **1810** on one side of the manifold **1800** are inserted into ports **1803** of one multi-layer flask **1801,** and the cannula **1811** on the other side of the manifold **1800** are inserted into ports **1804** of the other multi-layer flask **1802,** the two flasks can be joined together. Media and cells can be transferred from one vessel to the other through the manifold **1800,** so connected. For example, the vessel with cells to be distributed to another vessel (or several other vessels for cell proliferation) can be tilted upward as shown in Figure 18, and the cells can be transferred from a first vessel **1802** to a second vessel **1801** by gravity. In an alternative embodiment, a pump can be attached to a second set of ports **1830** to drive fluid from the first vessel **1802** to the second vessel **1801.** Or, a vacuum pump could be attached to the ports **1835** of the receiving vessel to pull fluid from the first vessel **1802** to the second vessel **1801.**

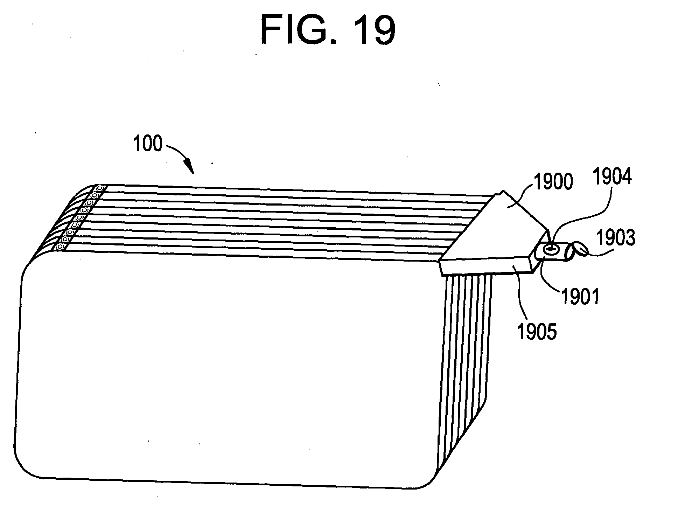

**Figure 19** illustrates an additional manifold, as shown in Figure 14, attached to a multi-layer flask. Figure 19 shows a multi-layer flask **100** with a manifold **1900** attached. The manifold **1900** has a manifold body **1905,** a necked opening **1901,** a cap **1903** and a valve **1904.** The manifold body **1905** couples with the multi-layer flask **100** through the ports (not shown) to allow fluid entering the necked opening **1901** to flow through the manifold **1900** and into the cell culture chambers of the multi layer flask **100.** The manifold **1900** may be permanently attached to the multi-layer flask, or it may be removable. The manifold **1900** may be disposable, and sterilizable. When the manifold **1900** is removed, the ports of the multi-layer flask may be covered by port covers, and access to the cell culture chambers of the multi-layer flask is limited. When the manifold **1900** is removed, the multi-layer flask has a regular rectangular or square footprint, allowing multiple multi-layer flasks to be placed into an enclosed space such as an incubator or a packing box without the need to accommodate an irregularly shaped manifold or opening.

**Figure 20** illustrates a reservoir or cell collection device **2020.** While the external manifold shown in Figure 20 is the embodiment shown in Figures 8, 9 and 10, any external manifold embodiment may be appropriate here. The external manifold **2000** has cannula **2010,** a manifold body **2005,** a valve **2004,** and a necked opening **2001.** The external manifold **2000** is structured and arranged to couple with a multi-layer cell culture container (not shown). The external manifold may be couples to an external liquid reservoir which can be a cell collection device or a culture media reservoir. Shown in Figure 20 is a cell collection device **2020.** The external manifold **2000** can be coupled to the cell collection device through a container cap **2015,** which is attached to the cell collection container **2020** by tubing **2002.** The container-end of the tubing **2025** slips into a perforation **2030** in the container **2020** when the container cap **2015** is screwed or snapped into place. In an alternative embodiment, the collection container can be preassembled with the tubing **2002** already attached to the container **2020.** The container **2020** has a second port **2040** for connecting to tubing **2041** which leads to a vacuum pump (not shown). When this assembly is connected to a multi-layer flask through the ports of the multi-layer flask, and a vacuum is provided to the container through the second port **2040**, the vacuum can cause liquid and, in some cases cells, to be removed from the multi-layer flask and deposited into the container **2020**. The container may be a sterile container, and the external manifold and tubing may be sterilized, allowing for the sterile removal of cells and fluid from a multi-layer cell culture flask to a container.

These processes can be performed in an automated setting. For example, an external manifold, connected to a sterile collection container as shown in Figure 20, may be manipulated robotically to couple to a multi-layer flask and remove the contents of the multi-layer flask. With this kind of robotic manipulation, human contact is reduced and the risks of contamination and spilling are reduced.

## Claims

1. A multi-layer cell culture device comprising:
at least three cell culture chambers, each cell culture chamber having at least one port;
at least two integral tracheal chambers; and,
a sliding port cover structured and arranged to engage with the at least one port of the at least three cell culture chambers of the multi-layer cell culture device and to provide an open or a closed port;
wherein the open or closed access is determined by slidingly engaging the port cover in an open position or a closed position in relation to the at least one port; and
wherein the port cover in the open position comprises a filter and the port cover in the closed position comprises a septum.

2. The multi-layer cell culture device of claim 1 wherein the sliding port cover is connected to the multi-layer cell culture device by a hinged connector.

3. The multi-layer cell culture device of claim 1 or 2 wherein the sliding port cover is integral with the multi-layer cell culture device.

## Patentansprüche

1. Mehrschichtige Zellkulturvorrichtung, umfassend:
zumindest drei Zellkulturkammern, wobei jede Zellkulturkammer über zumindest einen Anschluss verfügt;
zumindest zwei integrierte tracheale Kammern; und
eine verschiebbare Anschlussabdeckung, die so strukturiert und angeordnet ist, dass sie in den zumindest einen Anschluss der zumindest drei Zellkulturkammern der mehrschichtigen Zellkulturvorrichtung eingreift und einen offenen oder einen geschlossenen Anschluss bereitstellt;
wobei der offene oder geschlossene Zugang durch verschiebbares Eingreifen der Anschlussabdeckung in einer offenen Position oder einer geschlossenen Position in Bezug auf den zumindest einen Anschluss bestimmt wird; und
wobei die Anschlussabdeckung in der offenen Position einen Filter umfasst und die Anschlussabdeckung in der geschlossenen Position ein Septum umfasst.

2. Mehrschichtige Zellkulturvorrichtung nach Anspruch 1, wobei die verschiebbare Anschlussabdeckung durch ein drehbares Verbindungselement mit der mehrschichtigen Zellkulturvorrichtung verbunden ist.

3. Mehrschichtige Zellkulturvorrichtung nach Anspruch 1 oder 2, wobei die verschiebbare Anschlussabdeckung in die mehrschichtige Zellkulturvorrichtung integriert ist.

## Revendications

1. Dispositif de culture cellulaire multicouche, comprenant :
au moins trois chambres de culture cellulaire, chaque chambre de culture cellulaire comportant au moins un orifice ;
au moins deux chambres trachéales d'une seule pièce ; et
un couvercle d'orifice coulissant structuré et conçu pour coopérer avec l'au moins un orifice des au moins trois chambres de culture cellulaire du dispositif de culture cellulaire multicouche et pour fournir un orifice ouvert ou un orifice fermé ;
dans lequel l'accès ouverture ou fermé est déterminé par une coopération coulissante du couvercle d'orifice dans une position ouverte ou dans une position fermée par rapport à l'au moins un orifice ; et
dans lequel le couvercle d'orifice, dans la position ouverte, comprend un filtre et le couvercle d'orifice, dans la position fermée, comprend un septum.

2. Dispositif de culture cellulaire multicouche selon la revendication 1, dans lequel le couvercle d'orifice coulissant est relié au dispositif de culture cellulaire multicouche au moyen d'un dispositif de liaison articulé.

3. Dispositif de culture cellulaire multicouche selon la revendication 1 ou 2, dans lequel le couvercle d'orifice coulissant est d'un seul tenant avec le dispositif de culture cellulaire multicouche.
